# EUROPEAN PATENT APPLICATION

(11) **EP 1 512 971 A1**
(43) Date of publication of application: **09.03.2005**
(21) Application number: 03077769.2
(22) Date of filing: 04.09.2003
(51) Int. Cl.: G01N 33/52, G01N 31/22

(54) **A method for qualitative and/or quantitative detection of polyethylene glycols in biological fluids**

(71) Applicant: Université de Liège, 4020 Liège (BE)
(72) Inventor: Grandfils, Christian, Allée de la Chimie 3 4000 Liège (BE); Dandrifosse, Guy, Allée de la Chimie 3 4000 Liège (BE); Remacle, René, Allée de la Chimie 3 4000 Liège (BE); Barakat, Ibrahim, Allée de la Chimie 3 4000 Liège (BE)

(57) **Abstract**

The subject matter of the present invention relates to a method for qualitative and/or semi-quantitative and/or quantitative detection of polyethylene glycol (PEG) in biological fluids by addition of a reagent containing iodine, alkali metal iodide and an acid; and
a method for assessing the permeability of the intestine wherein two PEGs of different molecular weight, low molecular weight PEG (l.m.w. PEG) and high molecular weight PEG (h.m.w. PEG) are used as markers, wherein the l.m.w. PEG is able to cross the intestinal mucosa under physiological conditions and the h.m.w. PEG is essentially not able to cross the intestinal mucosa under physiological conditions, but is able to cross the intestinal mucosa under non-physiological, irregular conditions; and wherein h.m.w. and I.m.w. PEG concentrations are measured in a sample of urine from an individual after oral administration of h.m.w. PEG and I.m.w. PEG, wherein the differential index of h.m.w. PEG and I.m.w. PEG in the urine is calculated after determining the concentrations of h.m.w. PEG and l.m.w. PEG by using a reagent quantitative detection of polyethylene glycol (PEG) in biological fluids, the reagent containing iodine, alkali metal iodide and an acid; and
a kit for qualitative and/or semi-quantitative and/or quantitative detection of polyethylene glycol in biological fluids.

## Description

### Field of the invention

The present invention relates to a method for qualitative and/or semi-quantitative detection and/or quantitative detection of polyethylene glycols in biological fluids, a method for assessing the permeability of the intestine by detecting polyethylene glycols in biological fluids and a kit.

The subject matter encompasses a new method to measure the concentration of synthetic biocompatible macromolecules in biological fluids, particularly in order to evaluate the permeability of the intestine to macromolecules.

Polyethylene glycols are widely used as synthetic polymers in different fields, such as in the industry of food, agriculture, cosmetic, pharmaceutics and phytosanitary compounds as well as in medical applications.

The invention disclosed below is related to this class of polymers and the measurement of their concentrations in biological fluids.

For the present patent application, one of the main applications to measure polyethylene glycol concentration in biological fluids is the analysis of intestinal permeability, especially to macromolecules as explained below.

One essential function of the intestine is to work as a barrier in order to control the passage of molecules between intestinal lumen and blood compartments. Increase of this permeability does not only allow an anomalous passage of low molecular weight substances, but also, in certain circumstances, does permit antigens like proteins and viruses to cross the intestinal mucosa in high amounts. This passage may have pathological consequences like infections, inflammations, allergies and food intolerances. Up to now, commercially available permeability tests use only low molecular weight markers, such as lactulose, rhamnose, raffinose, cellobiose, or ⁵¹Cr-EDTA. However, these tests give only information on the intestinal permeability changes for low molecular weight products.

Polyethylene glycols (PEG, chemical abstract n° 25322-68-3) with a molecular weight in a range from 400 to 10,000 have also been reported as intestinal marker in the literature and some of them have been used for preliminary clinical tests. Polyethylene glycol (PEG) is defined as polyethylene glycol homopolymers having the generic formula H-[-O-CH₂-CH₂-]ₙ-OH or compounds containing one or several PEG sequences.

An increase of their intestinal permeability has been highlighted for example in Crohn disease and ulcerative colitis. However, the detection of these macromolecules in biological fluids like plasma or urine requires time-consuming analytical methodologies as well as expensive analytical instruments, limiting thereby their applications for routine analysis in clinics. Examples of these difficult analytical techniques are reported in the following references:
- Eur. J. Clin. Chem. Clin. Biochem. 32,813-820 (1994)
- J. Chromat. A800, 231-238 (1998)
- J. Chromat. 565 (1-2), 297-307 (1991)
- Analytical Letters, 20 (2), 293-301 (1987)

These techniques, which revealed to be difficult and time-consuming, were shortened by Hyden in 1955 [Hyden, S. The recovery of polyethylene glycol after passage through the digestive tract, Ann. R. Agric. Coll. Sweden 22, 411-424 (1955). As such designed, the method of Hyden requires to take a lot of precautions, due to the instability of the PEG-Ba²⁺-trichloroacetic acid emulsion. Malawer and Powell, [Malawer, S.J., Powell D.W.; An improved turbidimetric analysis of polyethylene glycol utilizing an emulsifier, Gastroenterology, 44, 250-256 (1967)], Buxton et al. [Buxton, T.B., Crockett, J.K., Morre, W.L., Moore, W.L., and Rissing J.P.; Protein precipitation by acetone for the analysis of polyethylene glycol in intestinal perfusion fluid, Gastroenterology, 76, 820-824 (1979)], Childs, [Childs, C.E., Microchemical J., 20, 190-192 (1975)], Ty [Ty, A. Microchemical J., 24, 287-290 (1979),], Skoog [Skoog, B., Vox Sang., 37, 345-349 (1979)], and Sims and Snape [Sims G.E.C. and Snape, T. J., Anal. Biochem., 107, 60-63 (1980)) have reported improvements of the assay, particularly by increasing its sensitivity. The methods of the state of art require the presence of barium to detect PEG. None of the methods of the state of art provide the possibility to differentially detect and/or measure polyethylene glycols of different molecular weights in case polyethylene glycols of different molecular weights are present in the same sample.

The physico-chemical nature of the interaction between iodine and PEG has been detailed by others [Hemalatha, S. et al.; Complexation of molecular iodine by linear poly (ethylene glycol), Spectroscopy Letter, 12, 535-541 (1979)] and Szalbocs [Szalbocs, E., Über lodkomplexe von hochpolymeren Polyethylenglycolen, Pharmazia, 39(4), 230-232 (1984)].

The object of the present invention is to provide a rapid method for differentially detecting and/or measuring of polyethylene glycols (PEG) in biological fluids, wherein in a given sample high molecular weight (h.m.w.) and low molecular weight (I.m.w.) PEG is present, and to provide a method for assessing the permeability of the intestine.

Surprisingly, we have now found a method for qualitative and/or semi-quantitative and/or quantitative detection of polyethylene glycols (PEG) in biological fluids by addition of a reagent containing iodine, alkali metal iodide and an acid and wherein barium ions are no more necessary to detect PEG by colorimetry. With this method according to the present invention it is possible to differentially detect and/or measure polyethylene glycols of different molecular weights, i.e. the claimed method allows the distinct determination of two different size PEG, when mixed in a sample, the one being of low molecular weight, the other of high molecular weight.

In a preferred embodiment of the method the biological fluid is pre-treated by ion exchange resins before the reagent is added. This purification allows a more accurate measurement of PEG, due to the removal of factors which might influence the reaction.

In a further preferred method the detection of polyethylene glycols is performed under conditions where essentially no barium ions are present in the reagent.

If PEG is present in the biological sample the mixing of the iodine reagent with the biological fluid induces a colour change of the solution. The optical density of the solution is related to the PEG concentration. Therefore, in a preferred embodiment of the method, after the addition of the reagent the presence of polyethylene glycol is determined by detecting a colour change. Preferably, this detection is performed by optical density measurement. For this measurement a wavelength between 500 and 700 nm is preferably used, most preferred a wavelength at 550 nm.

Polyethylene glycols (PEGs) to be detected by the method according to the present invention are defined as polyethylene glycol homopolymers having the generic formula:

H-[-O-CH₂-CH₂-]ₙ-OH

or compounds containing one or several PEG sequences. Preferably these compounds are covalently linked to one or several PEG sequences.

In particular, the reagent consists mainly of iodine, alkali metal iodide and an acid in an aqueous solution. The concentration of the acid is set between 10mM and 5M. As acid preferably acetic acid, boric acid or hydrochloric acid may be used, in particular acetic acid. As alkali metal iodide preferably potassium iodide may be used.

The method is suitable for determining polyethylene glycol concentrations, in a large range of molecular weights. But the method has also been adapted in order to determine the concentrations of both a low molecular weight PEG (I.m.w. PEG) and a high molecular weight PEG (h.m.w. PEG) present simultaneously in a biological fluid, i.e. an aqueous sample.

The method according to the present application can preferably be used for the analysis of biological fluids selected from the following group: urine, blood plasma, interstitial fluid, lymph, cerebrospinal fluid. In particular the final concentration in the reaction mixture of iodine for the measurement of PEG concentration is adjusted between 0.1 and 20 mM, preferably between 1 and 15 mM.

The object is also solved by a method for assessing the permeability of the intestine wherein two PEGs of different molecular weight, low molecular weight PEG (I.m.w. PEG) and high molecular weight PEG (h.m.w. PEG) are used as markers, wherein the I.m.w. PEG is able to cross the intestinal mucosa under physiological conditions and the h.m.w. PEG is essentially not able to cross the intestinal mucosa under physiological conditions, but is able to cross the intestinal mucosa under non-physiological, irregular conditions; and wherein h.m.w. and I.m.w. PEG concentrations are measured in a sample of urine from an individual after oral administration of h.m.w. PEG and I.m.w. PEG, wherein the differential index of h.m.w. PEG and I.m.w. PEG in the urine is calculated after determining the concentrations of h.m.w. PEG and I.m.w. PEG by using a reagent for quantitative detection of polyethylene glycol (PEG) in biological fluids, the reagent containing iodine, alkali metal iodide and an acid.

In a preferred embodiment, the method for assessing the permeability of the intestine comprises the following steps:
a) collecting a sample of urine from an individual after oral administration of high molecular weight polyethylene glycol (h.m.w. PEG) and low molecular weight polyethylene glycol (I.m.w. PEG);
b) for the determination of the h.m.w. PEG concentration:
   i) pretreating the urine sample by ion exchange resins;
   ii) adding a reagent containing iodine, alkali metal iodide and an acid to the pretreated urine sample,
      wherein the concentrations of iodine and the alkali metal iodide are adjusted in that way that essentially no colour reaction with the I.m.w. PEG but only with the h.m.w. PEG occurs;
   iii) determining optical density of the reaction mixture, eventually after a defined time;
c) for the determination of the I.m.w. PEG concentration:
   i) pretreating the urine sample by ion exchange resins;
   ii) adding a reagent containing iodine, alkali metal iodide and an acid to the pretreated urine sample, wherein the concentration of iodine and the alkali metal iodide is adjusted in that way that the colour reaction occurs with the I.m.w. PEG:

   i) determining optical density of the reaction mixture, eventually after a defined time.
By pretreatment, one means treatment of urine to eliminate compounds that may interfere in colorimetric measurements.
By ion exchange resins, one means cationic resin such as sulfonic acid resin, anionic resin such as quaterly ammonium resin or a mixture of them.

For the determination of PEG a urine sample with known h.m.w. and/or I.m.w. PEG concentration is prepared and treated in the same way as the biological sample as reference.

In another embodiment of the method, the method further comprises after the pretreatment of urine sample, a step for determining the h.m.w. or I.m.w.concentration by using a calibration curve obtained with the pretreated urine. Advantageously such additional steps allow an immediate evaluation of an unknown amount of PEG added to the pretreated urine.

In a preferred embodiment of the method the concentrations of h.mw. PEG and I.m.w. PEG in the urine sample are calculated from the optical density measurements, and then the ratio of h.m.w. PEG concentration on I.m.w. PEG concentration is determined as differential index. When the measurement is performed with urine as biological liquid this differential index indicates the permeability of the intestine.

The h.m.w. PEG and the I.m.w. PEG used in the biological sample, for example urine for receiving the calibration curve should have the same grade as the PEG administered to the patient.
In another embodiment of the method, the urine sample of step a) is divided in at least two portions before or after pre-treatment by ion exchange resins and the portions obtained are separately used in step b) and c).

The invention provides a method for the differential analysis of polyethylene glycols of different molecular weights in biological fluids. The inventive method is simple, sensitive, and rapid, allowing it to be carried out in a very short time and using non toxic environmental friendly reagents. This method also provides an intestinal permeability diagnostic methodology, as these molecules can pass through the gastro-intestinal tract and the kidney barrier. The information acquired from this diagnostic tool is of importance for the identification and the following of several intestinal diseases or other disorders, actually known or unknown, but related to a modification of intestinal permeability.

The method also provides a simple tool to investigate the efficiency of galenic forms designed to improve the bio-availability of drugs, particularly those concerned with the administration of macromolecular drugs, such as peptides, proteins, nucleic acids, and the likes.

Taking into account that PEG could be used as biomarkers in the early phase of drug evaluation in exploratory development, especially in order to state precisely the therapeutic dose of a new drug, to establish a dose-response relationship and to define a plausible relationship between biomarker, pharmacological and pathogenesis, the determination of concentrations of PEG of different molecular weights in biological fluids is also of high potential interest in the pharmaceutical industry.

The method according to the present invention is suitable for assessing the permeability of the intestinal barrier adopting two PEG of different molecular weight as biomarkers. For example PEG of low molecular weight (PEG (I.m.w.)) can be used as simple passive diffusion biomarker reference, i.e. able to cross the intestinal mucosa without interacting with a plasma membrane component. On the other hand PEG of high molecular weight (PEG (h.m.w.)) can be used as abnormal diffusion biomarker, i.e. almost unable to cross the intestinal mucosa in physiological conditions.

In addition, the determination of the differential index of a h.m.w. PEG concentration on a I.m.w. PEG concentration in an urine sample allows to take into account of possible physiological variations such as gastro-intestinal transit time, diuresis and so on.

In more details, the present invention is the description of an analytical technique which allows the measurement of PEG concentration in biological fluids. The invention is particularly of interest because the assay as designed is rapid, sensitive, non expensive, specific and applicable to a wide range of molecular weight of PEGs (between 200 and 200,000, preferably between 400 and at least 25,000). Due to the selectivity of the assay, it can be performed directly on urine or any other biological liquid as previously defined. Due to the simplicity of the test and the non-toxicity of the reagents required to perform it, the assay could be performed for example by a patient himself, in order to get at least a semi-quantitative evaluation of his intestinal permeability by the time.

As such available, this new diagnostic tool could find applications in clinics, in particular in order to better understand the origin, development, follow-up of the following clinical disorders: infections (due to prions, viruses, bacteria), allergies, food intolerance, intestinal disorders or diseases (Crohn disease, celiac disease, gastro-enteritis). Also other inflammation processes which could occur in systemic organs or tissues as a consequence of the altered intestinal passage of antigens may be studied. Furthermore by taking into account the simplicity and low cost of the assay, it could also be used by people in order to evaluate possible intestinal permeability changes during some stresses such as sport activities.

Application of the PEG assay may, however, not be limited only to the intestinal permeability test as reported above. In particular, it is also well known from the literature that PEGs are extensively used as additives for different applications, i.e. food, drugs, cosmetic, ointments. It is thus also highly desirable to be able to monitor the concentration of these different PEGs in biological fluids, in particular because side effects have been reported in some toxicity studies [Lifton, Lj., On the safety of "Golytely", Gastroenterology, 86, 214-216 (1984); Sturgill, B.C., Herold, D.A., Bruns, D.E., Renal tubular necrosis in burn patients treated with topical polyethylene glycol, Lab Invest, 46, 81A (1982); Herold, D.A., Rodehaver, G.T., Bellamy, W.T. Fitton, L.A., Bruns, D.E., Edlich, R.F.; Tocicity of topical polyethylene glycol; Toxicol. Appl. Pharmcol, 65, 329-335 (1982)].

In addition, the PEGs are frequently used as non-absorbable markers to study water movements in human and animal transport studies (Jacobson, E.D., Bondy, D.C., Broitman, S.A. et al.; Validity of polyethylene glycol in estimating intestinal water volume, Gastroenterology, 44, 761-767 (1963).

There are also other properties of PEG disclosed in the literature which justify the need to use the present invention to measure PEGs concentration in biological specimen. In particular, PEGs have been used in order to concentrate by dialysis or to purify proteins using respectively their hydration and complexation properties.

For the method preferably the reagent for the determination of h.m.w. PEG is used, which is prepared by the following steps:
a) iodine and alkali metal iodide are mixed under the solid state;
b) the mixture obtained in step a) is solubilized in water to obtain a final concentration of these two reactants iodine and alkali metal iodide of 50 mM and 230 mM, respectively;
c) use of the mixture of step b) as such, or
d) dilution of the mixture of step b) in an aqueous solution of alkali metal iodide by at least a factor of 2, preferably by at least a factor of 2.5, and most preferred by at least a factor of 2.78;
e) subsequent dilution in an acid.

The reagent for the determination of I.m.w. PEG is prepared by the following steps:
a) iodine and alkali metal iodide are mixed under the solid state;
b) the mixture obtained in step a) is solubilized in water to obtain a final concentration of these two reactants iodine and alkali metal iodide of 50 mM and 230 mM, respectively;
c) use of the mixture of step b) as such, or
d) dilution of the mixture of step b) in an aqueous solution of alkali metal iodide by at maximum a factor of 1.19;
e) subsequent dilution in an acid.

The concentration of the acid in the reagent used for method is between 10mM and 5M. Particularly, the acid may be selected from the following group: acetic acid, boric acid or hydrochloric acid, preferably acetic acid.

In order to measure the concentration of h.m.w. PEG without any interference of the presence of I.m.w. PEG the final concentration of iodine in the reaction mixture is adjusted to 0.1 - 5 mM, preferably to 1 - 5 mM, more preferred to 1 - 3.0 mM, even further preferred to 1.6 - 2.0 mM, more preferred to 1.8 - 2.0 mM, and most preferred to about 1.8 mM. For the measurement of I.m.w. PEG the final concentration of iodine in the reaction mixture is adjusted to higher than 5 mM, in particular it is adjusted to 5 - 20 mM, more preferred to 5 - 15 mM, even more preferred to 8 - 13 mM, and most preferred to 8.33 -12.5 mM.

For the method the relation of the concentration of iodine : alkali metal iodide in the reagent is in the range of 1 : 1 to 1 : 10, preferably in the range of 1 : 2 to 1 : 7, more preferred 1 : 3 to 1 : 6, more preferred 1 : 4 to 1 : 5, and most preferred about 1 : 4,6.

Furthermore the alkali metal iodide used shall be preferably potassium iodide.

After the addition of the reagent the optical density is measured using a wavelength between 500 and 700 nm. Particularly the optical density is measured at 550 nm.

The pretreatment of urine may be carried out by simple mixing of urine with ion exchange resins. Such ions exchange resins may be anionic, cationic or a mixture of them.
When a mixture of cation exchange resin (CE) and anion exchange (AE) resin is used, the volume ratio urine:CE:AE is in the range 0.1:1:1 to 10:1:1 ; preferably in the range 0.4:1.1. to 5:1:1 and most preferred 1:1:1.
After mixture urine and resins may be shaked during 2 to 50 minutes, preferably 15 minutes.

The h.m.w. PEG detected by the inventive method has a molecular weight in the range of 1,000 to 200,000, preferably between 4,000 and 25,000 and more preferred between 6,000 and 20,000.

The I.m.w. PEG used in this method has a mean molecular weight below 1,000, more preferred a mean molecular weight in the range of 300 to 500, and most preferred a mean molecular weight of about 400.

Using polyethylene glycols, especially a h.m.w and a I.m.w. PEG having these molecular weights, the status of the permeability of the intestine can be properly assessed.

It should be stressed that the I.m.w. PEG which is normally absorbed by a simple passive diffusion process into the intestinal mucosa, is used in the assessment of the intestinal permeability in order to avoid influences of gastro-intestinal transit time, diuresis and so on. Therefore the determination of the differential index of h.m.w. PEG concentration on I.m.w. PEG concentration in an urine sample allows to take into account of these possible physiological variations.

In order to give the best results in the measurement the final concentration of iodine in the reaction mixture may be varied depending on the exact molecular weight of the h.m.w. or I.m.w. PEG used in the assay. The iodine in the reaction mixture is adjusted to a final concentration of X [mM] depending on the molecular weight Y of the PEG according to the following table:

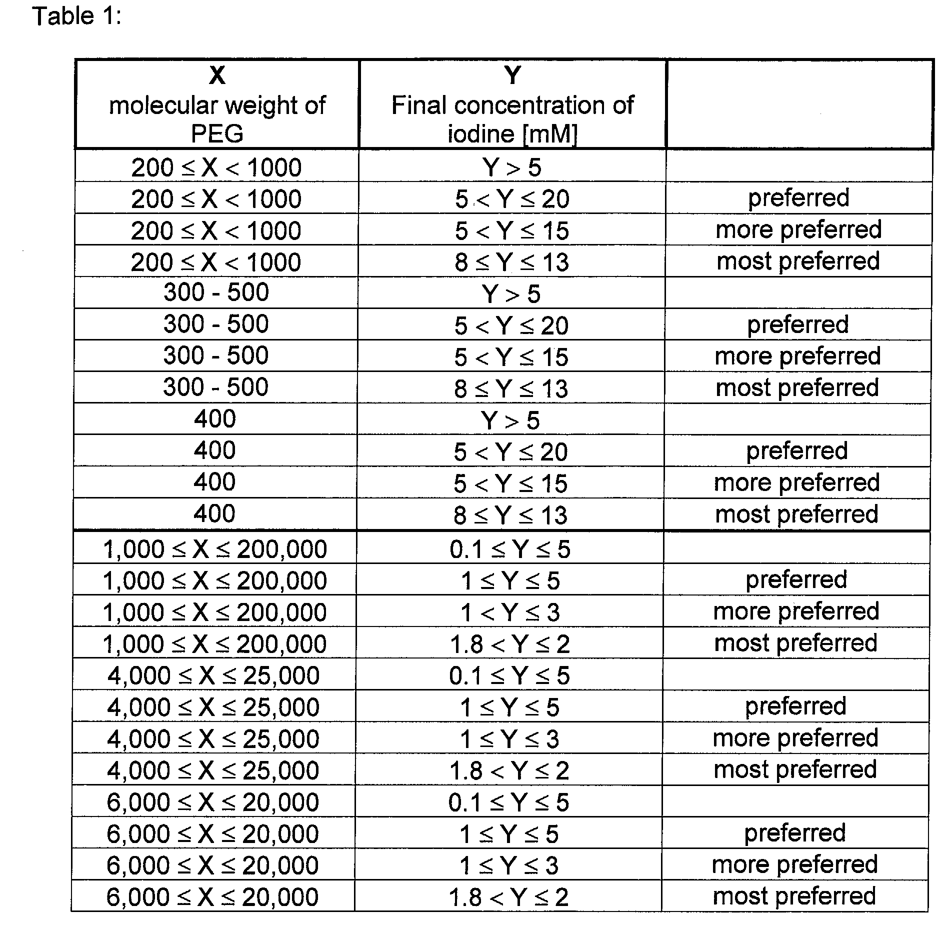

For the measurement of the permeability of the intestine a I.m.w. PEG preferably with a molecular weight of about 400 and a h.m.w. PEG with a molecular weight from 4,000 to 25,000 will be chosen. For example, h.m.w. PEGs with 6,000, 10,000 and 20,000, respectively have been used in the examples described below. Generally, for the determination of I.m.w. PEG the final concentration of iodine will preferably be in the range of between 5 and 15 mM, more preferred between 8 and 13. For the determination of the h.m.w. PEG the final concentration of iodine in the reaction mixture is adjusted to 0.1 - 5 mM, preferably to 1 - 5 mM, more preferred to 1 - 3.0 mM, even more preferred to 1.8 - 2.0 mM, and most preferred to about 1.8 mM. The lower the molecular weight of the PEG is, the higher the final concentration of the iodine has to be used since less molecules of iodine will be bound to each molecule of PEG and vice versa.

However, if h.m.w. PEG of a lower range (for example 6,000 PEG) is used for the determination of the h.m.w. PEG, the person skilled in the art will choose a final concentration of iodine which is not too close to 5 mM in order to avoid a colour reaction also with PEG 400. In this case a lower final concentration of 1.8 mM may be used in order to adjust the iodine concentration in that way that essentially no colour reaction with PEG 400 will occur.

The object is also solved by a kit for qualitative and/or semi-quantitative and/or quantitative detection of polyethylene glycols in biological fluids containing iodine, alkali metal iodide and an acid. In a preferred embodiment the alkali metal is potassium iodide. In a further embodiment the acid is either acetic acid, boric acid or hydrochloric acid, preferably acetic acid.

In a further preferred embodiment the kit contains iodine, alkali metal iodide and an acid. In a preferred embodiment the acid is selected from the following group: acetic acid, boric acid or hydrochloric acid may be contained in the kit, preferably acetic acid. As alkali metal iodide preferably potassium iodide is contained.

In a further preferred embodiment the kit contains PEG for use as positive control. In a particular preferred embodiment the kit contains a h.m.w. PEG and/or a I.m.w. PEG. The h.m.w. PEG has a mean molecular weight in the range of 1,000 to 200,000, preferably between 4,000 and 25,000 and more preferred between 6,000 and 20,000. The I.m.w. PEG used has a mean molecular weight below 1,000, more preferred a mean molecular weight in the range of 300 to 500, and most preferred a mean molecular weight of about 400.

### Description of the figures

The present invention will be explained in the figures, which show the results of the examples described below.
- Fig. 1: gives the optical densities of the reaction mixture obtained with three different urine samples containing different PEG 6,000 concentrations in presence of the reagent containing iodine, potassium iodide and acetic acid. The optical density was measured at 550 nm (n=3).
- Fig. 2: gives the optical densities of the reaction mixture obtained with three different urine samples containing different PEG 20,000 concentrations in presence of the reagent containing iodine, potassium iodide and acetic acid. The optical density was measured at 550 nm (n=3).
- Fig. 3: gives the optical densities of the reaction mixture obtained with three different urine samples containing different PEG 400 concentrations in presence of the reagent containing iodine, potassium iodide and acetic acid. The optical density was measured at 550 nm (n=3).
- Fig. 4: shows the measurement of PEG 35,000 in water (◆) and three different urine samples (▲, ■, ●), respectively, according to the methodology reported by Ty or Sims and Snape, but without purification step. The optical density was measured at 550 nm.

The following examples are given to illustrate the present invention. The scope of the invention, however, is not limited to the specific details of the examples.

### Examples

### Intestinal permeability measurements

According to the present invention the most preferred way to conduct the diagnostic test of the intestinal permeability assessment, is performed in the following manner.

A given dose of the PEG(s), between 0.1 and 50 g, but preferably between 1 and 10 g, will be taken by the patient per os. The molecular weight of the PEG(s) could be in the range of 400 to 20,000. This PEG(s) could be taken either under the form of a solution, or under a dry form where PEG(s) is (are) incorporated in a galenic form, such as a gelatin capsule. The urine of the patient will be collected for 6 up to 24 hours after the PEG oral administration. Urine can be collected in any adequate bottle, provided that the recipient can be adequately closed and chemically cleaned. Without any restriction to other possibilities, one adequate material for the collecting bottle is polyethylene. During collection, urine is kept in a fridge, for example at 4°C. If PEG concentration measurement does not occur immediately after collection, collected urine is conserved in a freezer, for example at -20°C. After thawing, urine sample may be centrifuged.

Next, the amount of PEG(s) recovered in urine is quantified in the following preferred way:

One volume of the collected urine is pretreated by one volume of a cation exchange resin (Amberlite 200 from Fluka) and one volume of an anion exchange resin (Amberlite IRA-900Cl from Supelco). The mixture of urine with resins is shaked for 15 minutes. Urine is then separated from resin by decantation and is called pretreated urine.
To a given volume of the pretreated urine a determined volume of an adequate reagent containing iodine and a given acid is added so that the final iodide concentration becomes 1.8mM for h.m.w. and 12.5 mM for I.m.w.PEG.
The addition of this iodine reagent is performed in such a way to assure an intimate and rapid mixing with the biological sample. As so prepared, the combination of the sample with the iodine reagent is called the reaction mixture. The iodine reagent is prepared according to the following steps:
- iodine and potassium iodide are simultaneously solubilized in water to obtain a concentration of these two reactants of 50 mM and 230 mM respectively,
- before the assay, this reagent is further diluted in an aqueous solution of potassium iodide . Dilution is carried out 2.78 times in potassium iodide 230mM for the preparation of 9mM potassium iodide used for h.m.w dosage The resulting mixture is subsequently diluted 2 times more in acetic acid 1M.

The mixing of the potassium iodine with PEG present in the biological sample induces a colour change of the solution whose optical density is related to the PEG concentration. The presence of a given PEG in urine can thus be visualized directly by the practitioner or eventually by the patient. But preferentially a precise quantification of PEG concentration in urine is desirable. A colorimetric analysis of the reaction mixture with reference to a calibration curve will allow to determine the PEG concentration in a range between 1 and at least 100 µg/ml of urine. In case of higher PEG concentration, a dilution of the urine sample is recommended.

The optical density is generally determined between 500 and 700 nm, preferentially at 550 nm.

### Example 1: Colorimetric calibration curve of PEG 6,000 in urine in the presence of fixed concentrations of PEG 400.

To a given volume of pretreated human urine are added PEG 400 and PEG 6,000 previously dissolved in water. The final concentrations of PEG 6,000 and PEG 400 in urine are ranged between 0 and 15 µg/ml and 50 and 200 µg/ml, respectively. To a given volume (0.8 ml) of this urine sample, 0.2 ml of an iodine reagent is added. The addition of this iodine reagent is performed in such a way to assure an intimate and rapid mixing with the biological sample. As so prepared, the combination of the sample with the iodine reagent is called the reaction mixture. The iodine reagent has been prepared according to the following steps:
- iodine and potassium iodide, previously mixed under the solid state have been simultaneously solubilized in water to obtain a final concentration of these two reactants of 50 mM and 230 mM respectively
- this reagent is further diluted at least by 2.78 in an aqueous solution of potassium iodide and subsequently 2 times more in acetic acid. The final concentration of the acetic acid in the dilution has to be ranged preferentially between 10mM and 5M.
- this iodine reagent can be stored during several months in a dark bottle before use.

For the determination of PEG 6,000 the final concentration of iodine in the reaction mixture in this example is 1.8 mM.

The mixing of the iodine reagent with PEG present in the biological sample induces a colour change of the solution with optical density is related to the polyether concentration. A colorimetric analysis of the reaction mixture allows to establish the calibration curve (eventually determined a given time after the mixing) which will allow to determine the PEG 6,000 concentration in a range between 1 and at least 30 µg/ml of urine. In case of higher PEG concentration, a dilution of the urine sample is recommended.

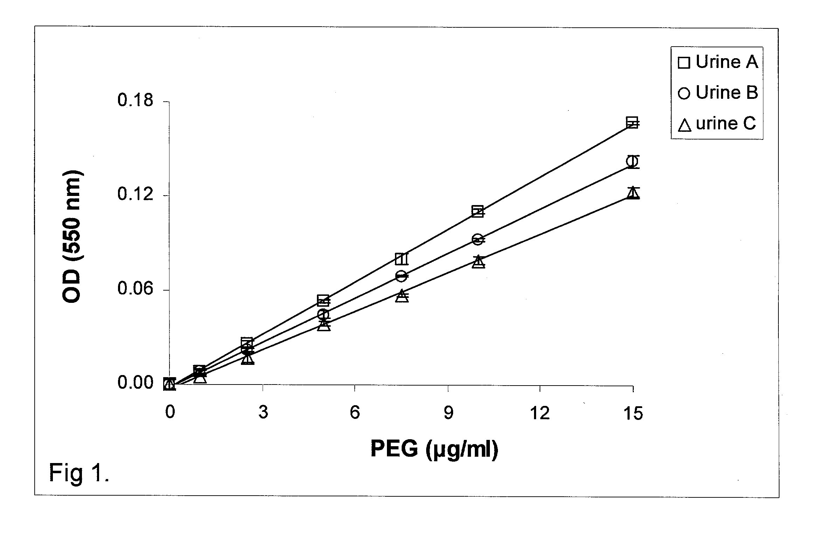

Figure 1 corresponds to typical calibration curves for PEG 6,000 obtained in these specific conditions. It shows the measurement of PEG 6,000 concentration in three different urine samples. The optical density was measured at 550 nm.

The measurement of PEG is very simple and based on a change of colour of the reaction mixture, which can be detected visually. It can be estimated by reference to a colour scale calibration curve.

### Example 2: Colorimetric calibration curve of PEG 20,000 in urine

To a given volume of pretreated human urine is added PEG 20,000 previously dissolved in water. The final concentration of PEG 20.000 in urine is ranged between 0 and 15 µg/ml. To a given volume (0.8 ml) of this urine sample, 0.2 ml of the iodine reagent is added. The addition of this iodine reagent is performed in such a way to assure an intimate and rapid mixing with the biological sample. As so prepared, the combination of the sample with the iodine reagent is called the reaction mixture. The iodine reagent has been prepared according to example 1.

For the determination of PEG 20,000 the final concentration of iodine in the reaction mixture in this example is 1.8 mM.

The mixing of the iodine reagent with PEG present in the biological sample induces a colour change of the solution whose optical density is related to the polyether concentration. A colorimetric analysis of the reaction mixture allows to establish the calibration curve (eventually determined a given time after the mixing) which will allow to determine the PEG 20,000 concentration in a range between 1 and at least 30 µg/ml or urine. In case of a higher PEG concentration, a dilution of the urine sample is recommended.

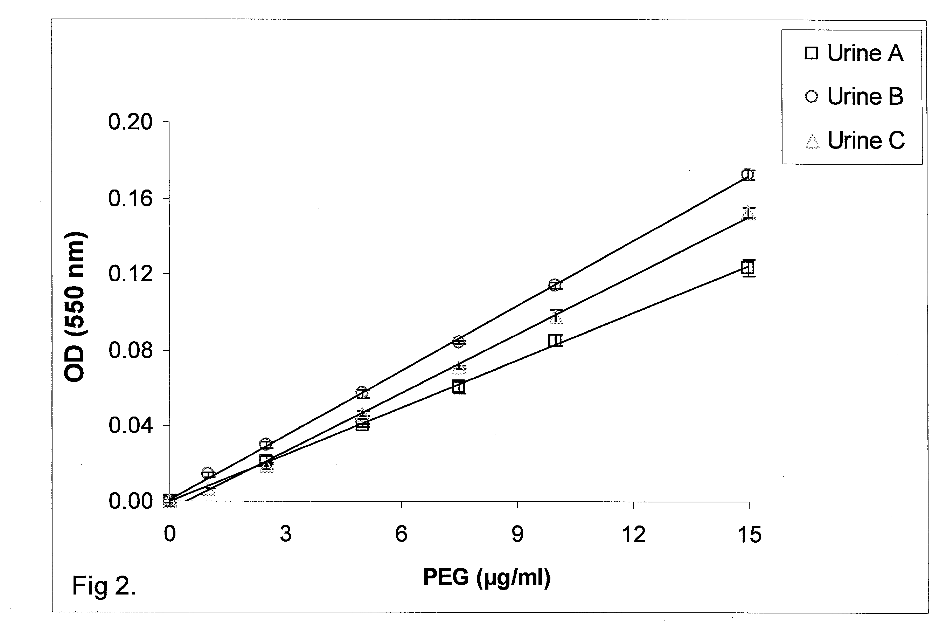

The optical density was measured at 550 nm. Figure 2 corresponds to typical calibration curves obtained in these specific conditions. The figure shows the measurement of PEG 20,000 in three different urine samples.

### Example 3: Colorimetric calibration curve of PEG 400 in urine in the presence of fixed concentrations of PEG 6,000.

To a given volume of pretreated human urine are added PEG 400 and PEG 6,000 previously dissolved in water. Final concentration of PEG 400 in urine is ranged between 0 and 140 µg/ml. To a given volume (0.8 ml) of this urine sample, iodine reagent is added (0.8 ml). The addition of this iodine reagent is performed in such a way to assure an intimate and rapid mixing with the biological sample. As so prepared, the combination of the sample with the iodine reagent is called the reaction mixture. The iodine reagent has been prepared according to the following steps:
- iodine and potassium iodide, previously mixed under the solid state have been simultaneously solubilized in water to obtain a final concentration of these two reactants of 50 mM and 230 mM, respectively.
- this reagent is used as such or further diluted by 1.19 in an aqueous solution of potassium iodide and subsequently 2 times more in acetic acid. The final concentration of the acetic acid in the dilution has to be ranged preferentially between 10mM and 5M.
- this iodine reagent can be stored during several months in a dark bottle before use.

For the determination of PEG 400 the final concentration of iodine in the reaction mixture in this example is 12.5 mM.

The mixing of the iodine reagent with PEG present in the biological sample induces a colour change of the solution whose optical density is related to the PEG concentration. A colorimetric analysis of the reaction mixture allows to establish the calibration curve (eventually determined a given time after the mixing) which will allow to determine the PEG 400 concentration in a range between 20 and at 140 µg/ml or urine. In case of higher PEG concentration, a dilution of the urine sample is recommend.

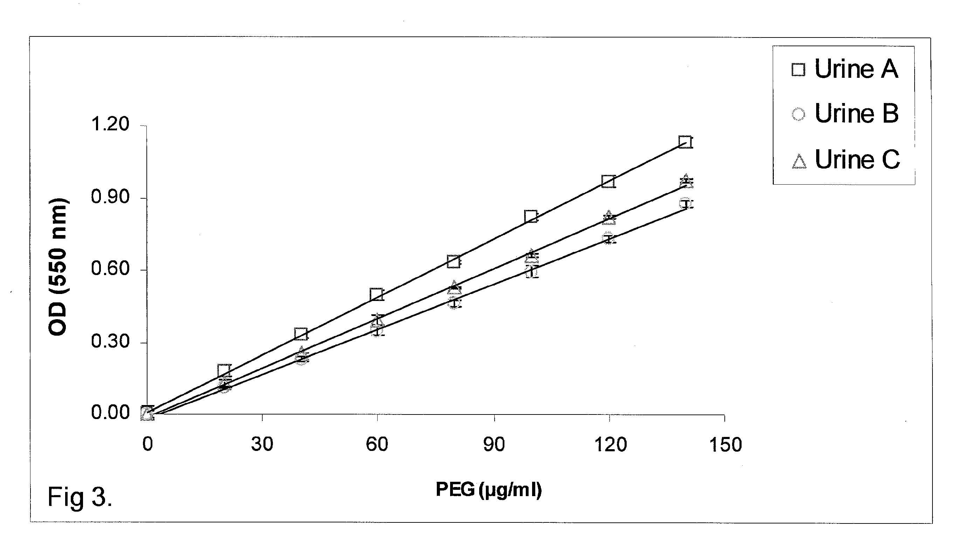

The optical density was measured at 550 nm. Figure 3 corresponds to typical calibration curves obtained for PEG 400 in the presence of fixed concentrations of PEG 6,000.

### Example 4: counter-example

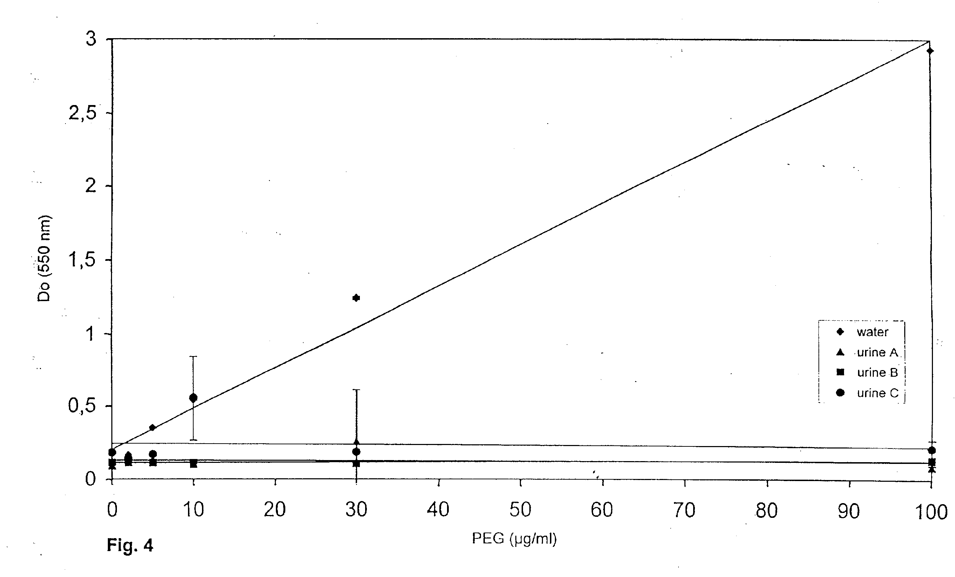

When the assay of PEG is performed according to the procedure reported by Ty or by Sims and Snape, but without any preliminary deproteinisation step, the presence of sulfate and phosphate ions in urine results in a precipitation of barium ions introduced by these authors to produce the colloid. As disclosed in figure 4, this precipitation of inorganic ions induces also the precipitation of PEG (in this example 35,000) (Figure 4: curves ▲, ■, ●) in the urine samples. No precipitiation occurs in water (Figure 4: curve ◆). This is explaining the absence of variation of the optical density in function of the PEG concentration when this latter is dissolved in the urine sample. The results are shown in figure 4. PEG 35,000 has been added to water (◆) and three different urine samples (▲, ■, ●), respectively.

### Example 5: Semi-quantitative evaluation of the intestinal permeability measurement carried out by the practitioner or eventually by the patient

The following example is given to explain the application of the ternary complex to analyze the intestinal permeability on a semi-quantitative basis by the practitioner or eventually by the patient.

According to the present invention, one of the most preferred ways to conduct the diagnostic test should be performed in the following manner:

A given dose of the low molecular weight PEG (between 0.1 and 50 g, but preferably between 1 and 10 g of PEG 200 -1000) and a high molecular weight PEG (between 0.1 and 50 g, but preferably between 1 and 10 g of PEG 6,000 - 20,000), could be taken by the patient either under the form of a solution or under a dry form where PEGs are incorporated in a galenic form, such as a gelatin capsule. The urine will be collected by the patient for 6 up to 24 hours after the PEG oral administration. During collection, urine is kept in a fridge, for example at 4°C. The urine will be submitted to a pretreatment, and mixed with a determined volume of an iodine reagent. The addition of this reagent is performed in such a way to assure an intimate and rapid mixing with the biological sample. In this case, the iodine reagent, made commercially available, will have the composition given in example 1. According to the range of molecular weight of PEG taken orally by the patient a significant modification of the colour intensity of the mixture appearing just after mixing will attest an alteration in intestinal permeability. The extent of colour intensity variation will be appreciated by the practitoner or eventually thepatient with the help of a given reference colour scale providing a semi-quantitative evaluation of the intestinal permeability modification.

### Example 6: Quantitative evaluation of the intestinal permeability measurement carried out in a clinical laboratory

The following example is given to explain the application of the ternary complex to analyze on a (semi)-quantitative basis the intestinal permeability using colorimetric analysis which can be applied for example in a clinical laboratory. According to the present invention a preferred way to conduct the diagnostic test should be performed in the following manner:

A given dose of the low molecular weight PEG (between 0.1 and 50 g, but preferably between 1 and 10 g of PEG 200 -1000) and a high molecular weight PEG (between 0.1 and 50 g, but preferably between 1 and 10 g of PEG 6,000 - 20,000), could be taken by the patient either under the form of a solution or under a dry form where PEGs are incorporated in a galenic form, such as a gelatin capsule. The urine will be collected by the patient for 6 up to 24 hours after the PEG oral administration. During collection, urine is kept in a fridge, for example at 4°C. If PEG concentration measurement does not occur immediately after collection, collected urine is conserved in a freezer, for example at -20°C. After thawing, urine sample may be centrifuged. The amount of both high and low molecular weight PEG recovered in urine could be quantified following the sequential steps described below:

### - step 1: quantification of the high molecular weight PEG:

To a given volume (0.8 ml) of a pretreated urine sample, 0.2 ml of iodine reagent is added. The addition of this reagent is performed in such a way to assure an intimate and rapid mixing with the biological sample. As so prepared, the combination of the sample with the iodine reagent is called the reaction mixture. The iodine reagent has been prepared according to the composition given in example 1.

The mixing of the iodine reagent with PEG present in the biological sample induces a colour change of the solution whose optical density is related to the PEG concentration. A colorimetric analysis of the reaction mixture with reference to a calibration curve (eventually prepared by using urine and detemined a given time after the mixing) will allow to determine the PEG concentration in a range between 1 and at least 30 µg/ml of urine. In case of higher PEG concentration, a dilution of the urine sample is recommended.

The optical density is generally determined between 500 and 700 nm, preferentially at 550 nm. In this specific concentration of iodine present in the reagent mixture, the presence of I.m.w. PEG 400 in the urine sample does not significantly interfere with analysis of the high molecular weight PEG.

### - step 2: quantification of I.m.w. PEG:

Once the h.m.w. PEG concentration is known, its absorption contribution can be subtracted from the analysis of I.m.w. PEG.

To a given volume (0.8 ml) of this urine sample, iodine reagent (0.8 ml) is added. The addition of this iodine reagent is performed in such a way to assure an intimate and rapid mixing with the biological sample. As so prepared, the combination of the sample with the iodine reagent is called the reaction mixture. The iodine reagent has been prepared according to example 3.

The mixing of the iodine reagent with PEG present in the biological sample induces a colour change of the solution whose optical density is related to the PEG concentration. A colorimetric analysis of the reaction mixture allows to establish the calibration curve (eventually prepared by using urineand determined a given time after the mixing) which will allow to determine the PEG 400 concentration in a range between 25 and 150 µg/ml of urine. In case of higher PEG concentration, a dilution of the urine sample is recommended. The optical density is generally determined between 500 and 700 nm, preferentially at 550 nm.

## Claims

1. A method for qualitative and/or semi-quantitative and/or quantitative detection of polyethylene glycols (PEG) in biological fluids by addition of a reagent containing iodine, alkali metal iodide and an acid.

2. The method according to claim 1, wherein the biological fluid is pretreated by ion exchange resins before the reagent is added.

3. The method according to claim 1 or 2, wherein the reagent is essentially barium free .

4. The method according to any one of claims 1 to 3, wherein after the addition of the reagent the presence of polyethylene glycol is determined by detecting a colour change.

5. The method according to any one of claims 1 to 4, wherein polyethylene glycols are represented by high molecular weight polyethylene glycol (h.m.w. PEG) and/or low molecular weight polyethylene glycol (I.m.w. PEG).

6. The method according to claim 5, wherein both h.m.w. PEG and/or I.m.w. PEG are present simultaneously in the biological fluid to be measured.

7. The method according to any one of claims 1 to 6, wherein the final concentration of iodine for the measurement of PEG is adjusted between 0.1 and 20 mM, preferably between 1 and 15 mM.

8. The method according to any one of claims 1 to 7, wherein the analysed biological fluids are selected from the group urine, blood plasma, interstitial fluid, lymph, cerebrospinal fluid.

9. A method for assessing the permeability of the intestine wherein two PEGs of different molecular weight, low molecular weight PEG (I.m.w. PEG) and high molecular weight PEG (h.m.w. PEG) are used as markers, wherein the I.m.w. PEG is able to cross the intestinal mucosa under physiological conditions and the h.m.w. PEG is essentially not able to cross the intestinal mucosa under physiological conditions, but is able to cross the intestinal mucosa under non-physiological, irregular conditions; and wherein h.m.w. and I.m.w. PEG are measured in a sample of urine from an individual after oral administration of h.m.w. PEG and I.m.w. PEG, wherein the differential index of h.m.w. PEG and I.m.w. PEG in the urine is calculated after determining the concentrations of h.m.w. PEG and I.m.w. PEG by using a reagent for quantitative detection of polyethylene glycol (PEG) in biological fluids, the reagent containing iodine, alkali metal iodide and an acid.

10. The method for assessing the permeability of the intestine according to claim 9, comprising the steps of:
a) collecting a sample of urine from an individual after oral administration of high molecular weight polyethylene glycol (h.m.w. PEG) and low molecular weight polyethylene glycol (I.m.w. PEG);
b) for the determination of the h.m.w. PEG concentration:
i) pretreating the urine sample by ions exchange resins
ii) adding a reagent containing iodine, alkali metal iodide and an acid to the pretreated urine sample ,
wherein the concentrations of iodine and the alkali metal iodide are adjusted in that way that essentially no colour reaction with the I.m.w. PEG but only with the h.m.w. PEG occurs;
iii) determining optical density of the reaction mixture, eventually after a defined time;
c) for the determination of the I.m.w. PEG concentration:
i) pretreating the urine sample by ion exchange resins;
ii) adding a reagent containing iodine, alkali metal iodide and an acid to the pretreated urine sample, wherein the concentration of iodine and the alkali metal iodide is adjusted in that way that the colour reaction occurs with the I.m.w. PEG :
iii) determining optical density of the reaction mixture, eventually after a defined time.

11. The method according to claim 9 or 10, wherein the concentrations of h.m.w. PEG and I.m.w. PEG in the urine sample are calculated from the data obtained by the optical density measurements, and then the ratio of h.m.w. PEG concentration on I.m.w. PEG concentration is determined as a differential index.

12. The method according to any one of claims 9 to 11, wherein the urine sample of step a) is divided in at least two portions before or after the ion exchange resin pre-treatment step and the portions obtained are separately used in steps b) and c).

13. The method according to any one of claims 1 to 12, wherein the concentration of the acid used is adjusted between 10mM and 5M.

14. The method according to any one of claims 1 to 13, wherein the acid used is selected from the following group: acetic acid, boric acid or hydrochloric acid, preferably acetic acid.

15. The method according to any one of claims 1 to 14, wherein the final concentration of iodine for the measurement of h.m.w. PEG is adjusted to 0.1 - 5 mM, preferably to 1 - 5 mM, more preferred to 1 - 3.0 mM, even further preferred to 1.6 - 2.0 mM, more preferred to 1.8 - 2.0 mM, and most preferred to about 1.8 mM.

16. The method according to any one of claims 1 to 15, wherein the final concentration of iodine for the measurement of I.m.w. PEG is adjusted to higher than 5 mM, preferably to 5 - 20 mM, more preferred to 5 - 15 mM, even more preferred to 8 - 13 mM, and most preferred to 8.33 - 12.5 mM.

17. The method according to any one of claims 1 to 16, wherein the relation of the concentration of iodine : alkali metal iodide in the reagent is in the range of 1 : 1 to 1 : 10, preferably in the range of 1 : 2 to 1 : 7, more preferred in the range of 1 : 3 to 1 : 6, even more preferred in the range of 1 : 4 to 1 : 5, and most preferred about 1 : 4,6.

18. The method according to any one of claims 1 to 17, wherein the alkali metal iodide used is potassium iodide.

19. The method according to any one of claims 1 to 18, wherein after the addition of the reagent the optical density is measured using a wavelength between 500 and 700 nm, preferably using a wavelength of 550 nm.

20. The method according to any one of claims 1 to 19, wherein the h.m.w. PEG used has a mean molecular weight in the range of 1,000 to 200,000, preferably between 4,000 and 25,000 and more preferred between 6,000 and 20,000.

21. The method according to any one of claims 1 to 20, wherein the I.m.w. PEG used has a mean molecular weight below 1,000, more preferred a mean molecular weight in the range of 300 to 500, and most preferred a mean molecular weight of about 400.

22. Kit for qualitative and/or semi-quantitative and/or quantitative detection of polyethylene glycol in biological fluids containing:
i) iodine
ii) alkali metal iodide
iii) an acid.

23. Kit according to claim 22, wherein the alkali metal iodide is potassium iodide.

24. Kit according to claim 23 or 22, wherein the acid is selected from the following group: acetic acid, boric acid or hydrochloric acid, preferably acetic acid.
